# EUROPEAN PATENT SPECIFICATION

(11) **EP 2 506 779 B1**
(45) Date of publication and mention of the grant of the patent: **20.11.2013**
(21) Application number: 10803517.1
(22) Date of filing: 27.10.2010
(51) Int. Cl.: A61B 17/128, A61B 17/12, A61B 17/10, A61B 17/122

(54) **A LIGATION DEVICE FOR SURGICAL INTERVENTION**
LIGATURVORRICHTUNG FÜR CHIRURGISCHE EINGRIFFE
DISPOSITIF DE LIGATURE POUR UNE INTERVENTION CHIRURGICALE

(30) Priority: 02.12.2009 IN KO14102009
(43) Date of publication of application: 10.10.2012
(73) Proprietor: Johnson and Johnson Ltd., Kolkata 700 016 (IN)
(72) Inventor: CHARUDATTA, Chandrakant Aradhye, Mumbai 400 057 Maharashtra (IN); DHANURAJ, Shiva Shetty, Jersey City, NJ 07302 (US); MURTY, Natarajan Vyakarnam, Springfield, NJ 07081 (US); YUFU, Li, Bridgewater, NJ 08807 (US)
(74) Representative: Tunstall, Christopher Stephen
(86) International application number: PCT/IN2010/000698
(87) International publication number: WO 2011/067778

(56) References cited:
- WO-A1-93/22973
- WO-A2-01/17448
- DE-U1- 29 616 065
- US-A- 4 856 518
- US-A- 5 383 883
- US-A- 5 797 931
- US-A1- 2006 190 013

## Description

### FIELD OF THE INVENTION

The present invention relates to an improved ligation device adaptable for surgical intervention.

### BACKGROUND OF INVENTION

Surgical intervention provides cure for many ailments and conditions which are not easily treatable by other medical techniques like drugs, radiation and the like.

Typically a surgical intervention involves locating the diseased/abnormal body part/organ, separating the located part and treating the body part/organ. The most important outcome expected from any surgical intervention apart from curing the ailments is minimization of the blood loss which requires ligation of vessels.

Hence, ligation device is an essential requirement for any surgical treatment to prevent blood loss. A typical surgical intervention involves cutting through the mesentery and separating the organs from connecting tissue, or manipulating a tissue which encounter pressure of blood vessels and other ducts. In most of cases a ligation device is used to ligate a vessel before cutting through it to prevent loss of vital fluids. Various techniques and corresponding devices for ligation of the vessel/ducts are known such as mechanical means, energy-based means for sealing of the vessel, manual means, and the like.

A device for duct closure/clamping is one of the most important means for almost every type of open and Laparoscopic surgical intervention.

Duct closure/clamping is imperative in hemostasis (all blood vessels), duct ligations (tubular structures inside living body carrying solid, liquid, gaseous materials), industrial applications (tubular structures carrying solid, semi-solid, liquid, gaseous material) etc.

There are various devices to achieve duct closure/clamping, following are the most common ones:
- Energy based : Electric Cautery, RF, Ultrasound, welding etc.
- Manual : Suture Ligation
- Mechanical : Ligation Clips, Rivets, clamps etc.

Suture ligation is one of the conventional and most widely known technique for ligating a blood vessel, which however constitutes a manually-operated technique. Suture ligation involves locating the point of ligation and tying a suture knot on the vessel/duct at the identified location. Suture ligation is a highly skilled based and is time consuming. Moreover, in the conventional method, two operators are required for respectively nipping the cut end of the blood vessel manually and at a faster pace to stop bleeding, and ligaturing it with the suture. Also, when the cut end of the blood vessel is beyond the reach of the operator's hand, it is impossible to ligature the cut end of the blood vessel. In addition, the form of the suture material presents additional problems for example, handling the thread-needs appropriately. Further, an additional step of trimming the thread is involved.

Hence, alternatives for suture ligation were developed. Mechanical means for example, clip, rivets, clamps provides a faster and less skill-based technique for ligation of vessels/ducts. However, it has its won disadvantages. Clip ligation involves applying a non-uniform closure force at the time of ligation, causing the vessel/duct to squeeze out from the clip, resulting in clip slippage and failure of the ligation. Further, metal clips that are used widely provide additional disadvantages specially when used in head and neck surgeries.

Other ligation means include thermal and electrosurgical sealing wherein thermal or electrical energy is applied across the vessel to fuse the vessel layers to together forming a seal. However, the use of energy causes additional problems such as patient burns, charring of surrounding tissues, thermal spread to close by vessels and vital organs, and the like.

Document US 5 797 931 A discloses a vessel ligation device comprising: a handle having a trigger and a heater switch; a force transmission driver connected to the trigger; an elongated shaft; and a pair of jaws distally disposed on the longitudinal shaft, wherein said pair of jaws comprises at least one heater and accommodates at least two pairs of precut materials which by thermal application of heat by the heaters fuse the pre-cut materials together around the vessel. This document is the closest prior art to the subject matter of independent claim 1.

**The disadvantages of the prior art devices can be summarized as under:**
- Slippage-Current metallic clips slips off, because of lack of positive locking and non uniform pressure distribution along the width of the vessel/duct,
- Difficulty in use of a plurality of clips when requited to ensure secured ligation,
- Hindrance to the post surgical imaging-being metal clips, the surfaces of the clips scatter the rays and deteriorate the image quality to a large extent.
- Suture ligation is a cumbersome and time consuming method
- Energy based hemostasis devices are not effective to take care of the large size vessels, and
- In case of energy based hemostasis devices, there is no physical evidence of vessel ligation which is very important in case of large size vessels.

### OBJECTS OF THE INVENTION

It is therefore an object of the invention to propose an improved ligation device adaptable for surgical intervention, which eliminates the disadvantages of prior art.

Another object of the invention is to propose an improved ligation device adaptable for surgical intervention, which has an in-built means for forming ligature in-situ.

A still another object of the invention is to propose an improved ligation device adaptable for surgical intervention, which is capable of securing large size vessels/ducts, and achieving a uniform pressure distribution along the ducts.

Yet another object of the invention is to propose an improved ligation device adaptable for surgical intervention, which ensures a good quality of post-surgical images due to use of fusable raw material for forming ligature.

A further object of the invention is to propose an improved ligation device adaptable for surgical intervention, which causes less damage to the tissue properties.

A still further object of the invention is to propose an improved ligation device adaptable for surgical intervention, which is enabled to make use of bioabsorbable raw materials having melting point below 100°C and can be heated to a semi-solid and fused around a vessel to be litigated.

### SUMMARY OF THE INVENTION

Accordingly, the present invention provides a ligation device adaptable for surgical intervention as defined in appended claim 1.

Preferred embodiments are defined in appended claims 2 to 7.

### BRIEF DESCRIPTION OF THE ACCOMPANYING DRAWINGS

- Figure 1 -: shows a pictorial view of an embodiment of the ligation device adaptable for surgical intervention,
- Figure 2 -: shows a frontal view of the device of Figure-1 with the details of the jaws accommodating precut materials,
- Figure 3 -: depicts a flow-chart describing the sequential steps of operating of the device of Figure-1,
- Figure 4 -: shows a pictorial view of an embodiment of the inventive device,
- Figure 5 -: shows a pictorial view of an embodiment of the inventive device.

### DETAILED DESCRIPTION OF THE INVENTION

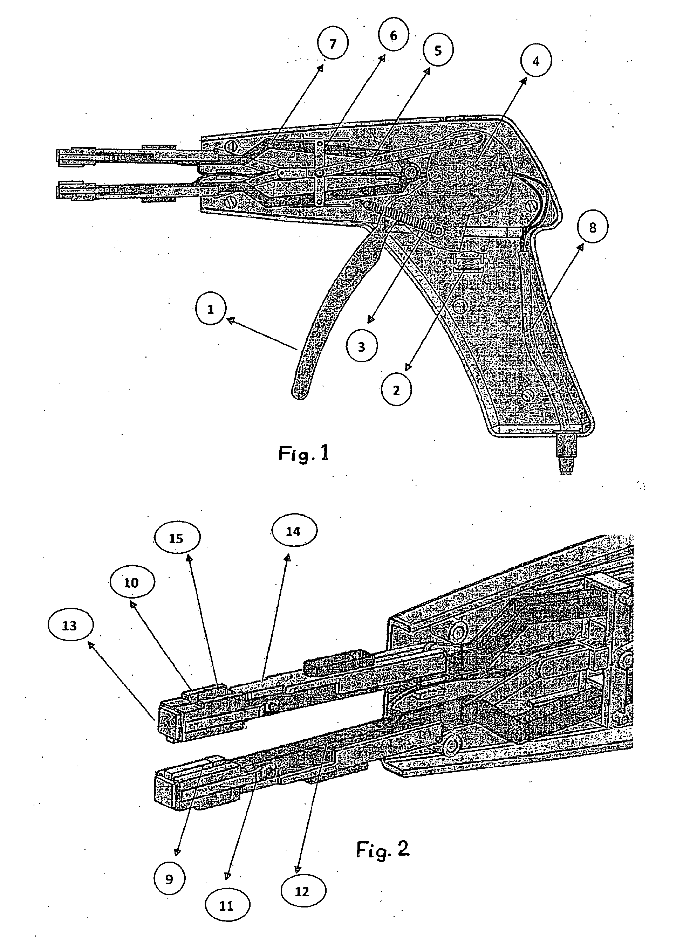

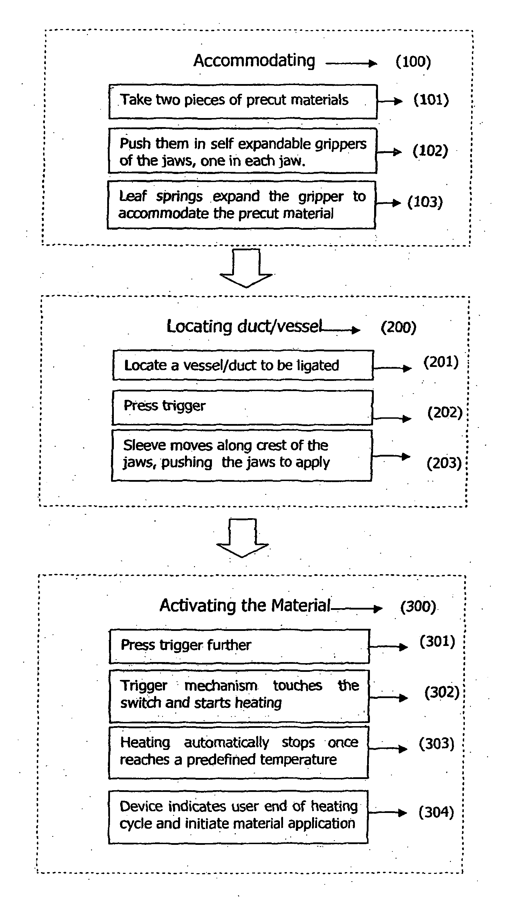

As shown in Figures 1 and 2, the ligation device comprises a shaped configuration with a handle portion and a body portion, the body portion constitutes a substantially parallel part attached to the handle portion at an angle of about 45°. The body portion is covered by an outer casing (16). A trigger (1) is integrally connected to a wheel (4) through a spring (3) such that the trigger (1) when is pulled causes the wheel (4) starts rotating. The rotational movement of the wheel (4) is translated to a longitudinal motion via a crank (5) connected to the wheel (4). A pair of crest (7) at one end connected to a pair of jaws (12), the other end of the pair of crest (7) is connected to the wheel (4). The handle portion is provided with a heater switch (2). A sleeve (6) is attached to the crests (7) which is further connected to the cranks (5). A Provision (8) is made for supply of electric power to at least one heater (13). When the trigger (1) is pressed, the sleeve (6) is caused to move along the crest (7) of the jaws (12). A further pressing of the trigger (1) allows the trigger mechanism to activate the heater switch (2) causing the heaters (13) to start functioning. A pair of self-expandable grippers (10) capable of accommodating at least two pieces of precut materials (9) to form ligature can be placed on the frontal portion of the jaws (12), the precut materials (9) on activation of the heaters (13) get heated to a predetermined temperature to form the ligature and fused over the vessel/duct. The grippers (10) comprise corresponding pusher element (14) and wedge element (15), the pusher element (14) is enabled to move forward by the motion of the crank (5) into the wedge element (15).

A pair of leaf springs (13) causes the gripper (10) to expand and eject the formed ligature from over the located vessel/duct after completion of the intervention.

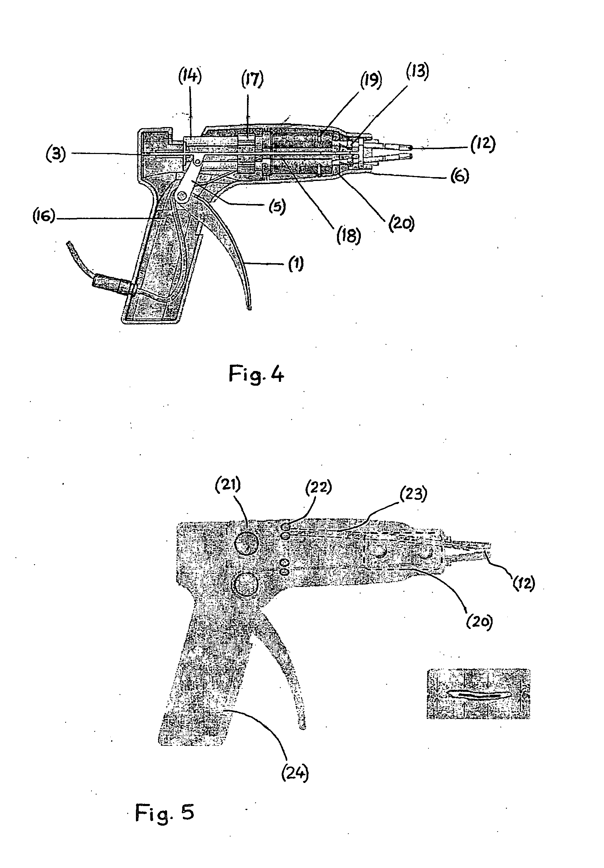

In the preferred embodiment of the device, the materials used for forming ligature is in the form of precut material. The intended result can be also achieved by using material in different forms as under :
Rod form-Material used is in the rod form (billet). As shown in figure 4, the embodiment of the device comprises an activation chamber (20) which converts the solid rod into semi-solid state and feeds it to the jaws which are provided with mold cavity. Accordingly, the ligature is formed in required shape which is applied around a vessel. A feeder (17) receives the material in rod form, and the heater (13) is externally provided with an insulator (19). The device is provided with an outer casing (16). The ligature may be formed for example, in the shape of a clip produced from polymeric material which may contain drug/additives to reduce post-surgical infections, surgical adhesions, and other post-surgical complications.

The polymers used to prepare the ligating film described herein are biodegradable and biocompatible. The biodegradable polymers readily break down into small segments when exposed to moist body tissue. The segments then either are absorbed by the body, or passed by the body. More particularly, the biodegraded segments do not elicit permanent chronic foreign body reaction, because they are absorbed by the body or passed from the body, such that no permanent trace or residual of the segment is retained by the body.

Polymers having melting point less than 100°C are preferred. Examples of suitable biocompatible, biodegradable polymers include polymers selected from the group consisting of aliphatic polyesters, poly(amino acids), copoly(ether-esters), polyalkylene oxalates, polyamides, poly(iminocarbonates), polyorthoesters, polyoxaesters, polyamidoesters, polyoxaesters containing amine groups, poly(anhydrides), polyphosphazenes, biomolecules and blends thereof.

For the purpose of this invention aliphatic polyesters include, but are not limited to homopolymers and copolymers of epsilon-caprolactone, where suitable monomers to prepare the copolymers of epsilon-caprolactone includes, but are not limited to lactic acid, d-, 1- and meso lactide), glycolide (including glycolic acid), p-dioxanone (1,4-dioxan-2-one), trimethylene carbonate (1,3-dioxan-2-one), alkyl derivatives of trimethylene carbonate. In particular, elastomeric copolymers of epsilon cparolactone may be useful. Suitable bioabsorbable, biocompatible elastomeric copolymers include but are not limited to copolymers of epsilon-caprolactone and glycolide (preferably having a mole ratio of epsilon-caprolactone to glycolide of from about 30:70 to about 70:30, preferably 35:65 to about 65:35, and more preferably 45:55 to 35:65); elastomeric copolymers of epsilon-caprolactone and lactide, including L-lactide, D-lactide blends thereof or lactic acid copolymers (preferably having a mole ratio of epsilon-caprolactone to lactide of from about 35:65 to about 65:35 and more preferably 45:55 to 30:70) elastomeric copolymers of p-dioxanone (1,4-dioxan-2-one) and lactide including L-lactide, D-lacide and lactic acid (preferably having a mole ratio of p-dioxanone to lactide of from about 40:60 to about 60:40); elastomeric copolymers of epsilon-caprolactone and p-dioxanone (preferably having a mole ratio of epsilon-caprolactone to p-dioxanone of from about 30:70 to about 70:30); elastomeric copolymers of p-dioxanone and trimethylene carbonate (preferably having a mole ratio of p-dioxanone to trimethylene carbonate of from about 30:70 to about 70:30); elastomeric copolymers of trimethylene carbonate and glycolide (preferably having a mole ratio of trimethylene carbonate to glycolide of from about 30:70 to about 70:30); elastomeric copolymer of trimethylene carbonate and lactide including L-lactide, D-lactide, blends thereof or lactic acid copolymers (preferably having a mole ratio of trimethylene carbonate to lactide of from about 30:70 to about 70:30) and blends thereof. In one embodiment, the elastomeric copolymer is a copolymer of glycolide and epsilon-caprolactone. In another embodiment, the elastomeric copolymer is a copolymer of lactide and epsilon-caprolactone. One of skill in the art would be able to identify suitable polymers as described above having a melting temperature of less than 100°C.

According to an embodiment of the invention as shown in figure 5, the device is provided with continuous Strip form-Material in the form of a spool of continuous strip (21) which is advanced to the jaws (12) over at least two rollers (22). The activation chamber (20) converts the material strip into semi-solid state and feeds to the jaws (12) which are provided with a mold cavity which allows forming the material in required shape and application of the ligature around a vessel. A guiding track (23) enables the continuous strip to move towards the jaws (12). A battery (24) is provided to supplement the mains power when necessary.

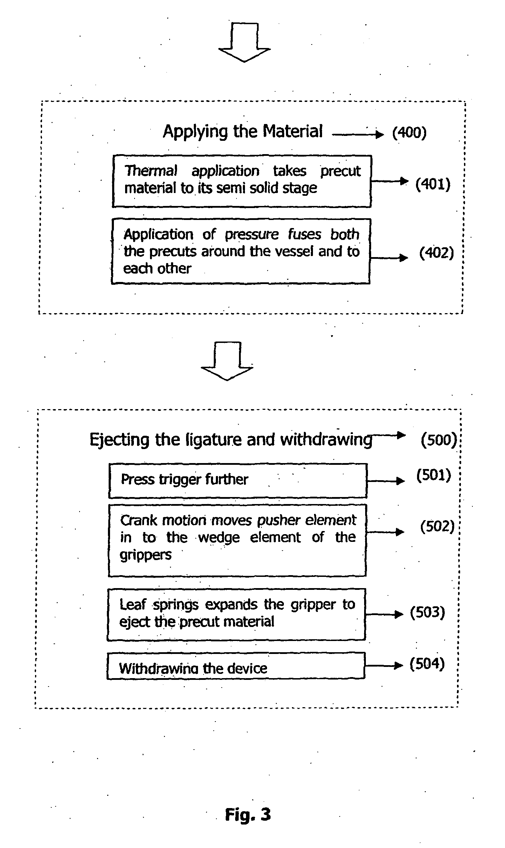

As shown in figure 3, the exemplary operating method of the device is described. There involves five main steps while the device is in active stage during a process of surgical intervention. The main steps respectively are, accommodating the ligator material (100), locating the duct/vessel (200), activating the material (300), applying the material (400), ejecting the ligature, and withdrawing the device (500).

The vessel to be ligated is located and held in the jaws (12). Pushing the trigger (1) which pushes the sleeve (6) along the jaws (12). Sleeve movement in forward direction brings the jaws (12) close to each other. When the sleeve (6) reaches at the crest of the jaws (12), pressure at the tip of the jaws (12) (pressure on vessel) reaches its maximum limit.

The trigger (1) is pressed further till it touches the heater switch (2). As soon as the trigger (1) pushes the switch (2), the heater (13) gets activated and heat the precut (9) to a required temperature and once the required temperature is achieved, power supply to the heater (13) is cut off automatically.

The trigger (1) is pressed further. As the trigger (1) is pressed, a pusher (14) moves forward and starts to engage with an wedge (15) of the gripper (10). The shape of the gripper (10) is configured in such a way that only towards the end of the trigger stroke (extreme condition of the trigger) the wedge (15) gets spring opened (approximately by 0.5mm) thus allowing ejection of the precut (9) from the jaws (12).

In gist, the sequential exemplary steps performed in operating the device during a surgical intervention is shown in Figure 3, are as under:
Providing (101) at least two pieces of precut materials for forming ligature; expanding at least two grippers using two leaf springs (103); placing the precut materials on the grippers (102), locating (201) the vessel/duct identified for ligation via the jaws; pressing (202) the trigger causing the sleeve to move along the crest of the jaws; pushing (203) the jaws to apply pressure; pressing the trigger further till such time the trigger touches the heating switch to activate the heater (301, 302); automatically stopping the heater on reaching predefined temperature (303); initiating material application on receipt of indication respecting end of the heating cycle (304); converting the material to a semi-solid stage through thermal application (401); fusing both the precuts to each other around the vessel through application of pressure (402); moving the pressure element into the wedge element of the grippers by further pressing the trigger leading to imparting a crank motion (502, 503); ejecting the precut materials via the leaf springs (504); and withdrawing the device (505).

## Claims

1. A ligation device for surgical intervention comprising:
- a shaped body with an outer casing (16), the body comprises a handle portion and a body portion;
- a rotatable wheel (4) attached to a shaft (5) disposed inside the body portion, the wheel (4) being integrally connected to a trigger (1) interposed adjacent the handle portion, the trigger (1) being manually operable against the tension force of a spring (3);
- a pair of jaws (12) distally disposed on the bod portion, and having a pair of crests (7) at their proximal ends, the pair of jaws (12) being connected at their proximal end to the body portion;
- a sleeve (6) connected to the shaft (5) and movable along the crests (7) on application of a pulling force on the trigger (1), a heater switch (2) provided on the handle portion which on further application of force on the trigger (10) gets activated causing at least one heater (13) to commence a heating function; and
- a gripping system comprising: a pair of grippers (10), a pusher element (14) and a wedge element (15), the pusher element (14) being configured to move along the jaw (12) by the action of a force applied to the trigger (1) against the action of at least one leaf spring (11), the grippers (10) being configured to accommodate at least two precut materials (9) which on thermal application by the at least one heater (13) form a ligator, and the ligator getting fused and deployed through application of further pressure on the trigger.

2. The device as claimed in claim 1, wherein when the ligator being formed of billets in rod form, the device further comprising an activation chamber (20) for converting the solid rod to a semi-solid state.

3. The device as claimed in claim 1 or 2, wherein a cavity is provided on the jaws (12), and wherein an insulator (19) is provided around the heater (13).

4. The device as claimed in claim 1, wherein when the ligator is formed of a continuous strip material (21), at least two rollers (22) are provided inside the body portion to enable advancement of the continuous strip material (21).

5. The device as claimed in claim 1 or 4, wherein the jaws (12) are provided with a cavity to allow formation of the ligator out of the strip material (21) subsequent to heating by the heaters (13).

6. The device as claimed in any of the preceding claims, comprising an electric supply means (8) for supply of power to the at least one heater (13).

7. The device as claimed in any of the preceding claims, comprising a battery (24) disposed inside the handle portion.

## Patentansprüche

1. Ligaturvorrichtung für chirurgische Eingriffe, umfassend
- einen geformten Körper mit einem Außengehäuse (16), wobei der Körper einen Griffabschnitt und einen Körperabschnitt umfasst,
- ein drehbares Rad (4), das an einer im Körperabschnitt angeordneten Welle (5) angebracht und integral mit einem in der Nähe des Griffabschnitts eingefügten Abzug (1) verbunden ist, der manuell gegen die Spannkraft einer Feder (3) betätigt werden kann,
- ein Paar Backen (12), die distal am Körperabschnitt angeordnet sind und ein Paar Kuppen (7) an ihren proximalen Enden haben, wobei das Paar Backen (12) an ihrem proximalen Ende mit dem Körperabschnitt verbunden sind,
- eine Hülse (6), die mit der Welle (5) verbunden und bei Ausübung einer Zugkraft auf den Abzug (1) entlang den Kuppen (7) bewegbar ist, einen Heizungsschalter (2), der am Griffabschnitt vorgesehen ist und bei weiterer Ausübung von Kraft auf den Abzug (10) betätigt wird, wodurch veranlasst wird, dass mindestens eine Heizung (13) eine Heizfunktion aufnimmt, und
- ein Greifsystem, das Folgendes umfasst: Greifer (10), ein Schieberelement (14) und ein Keilelement (15), wobei das Schieberelement (14) so konfiguriert ist, dass es sich durch die Wirkung einer gegen die Wirkung mindestens einer Blattfeder (11) auf den Abzug (1) ausgeübten Kraft entlang der Backe (12) bewegt, wobei die Greifer (10) so konfiguriert sind, dass sie mindestens zwei vorgeschnittene Materialien (9) aufnehmen, die bei der Beaufschlagung mit Wärme durch die mindestens eine Heizung (13) einen Ligator bilden, der durch die Ausübung weiteren Drucks auf den Abzug verschmolzen und ausgebracht wird.

2. Vorrichtung nach Anspruch 1, wobei die Vorrichtung, wenn der Ligator aus Barren in Stabform gebildet wird, ferner eine Aktivierungskammer (20) zur Umwandlung des festen Stabs in einen halbfesten Zustand umfasst.

3. Vorrichtung nach Anspruch 1 oder 2, wobei ein Hohlraum an den Backen (12) vorgesehen ist und wobei eine Isolierung (19) um die Heizung (13) vorgesehen ist.

4. Vorrichtung nach Anspruch 1, wobei, wenn der Ligator aus einem durchgehenden Streifenmaterial (21) gebildet ist, mindestens zwei Rollen (22) im Körperabschnitt vorgesehen sind, um das Vorschieben des durchgehenden Streifenmaterials (21) zu ermöglichen.

5. Vorrichtung nach Anspruch 1 oder 4, wobei die Backen (12) mit einem Hohlraum versehen sind, um die Bildung des Ligators aus dem Streifenmaterial (21) nach dem Erhitzen durch die Heizungen (13) zu gestatten.

6. Vorrichtung nach einem der vorhergehenden Ansprüche, umfassend ein Mittel (8) zur elektrischen Versorgung, um die mindestens eine Heizung (13) mit Energie zu versorgen.

7. Vorrichtung nach einem der vorhergehenden Ansprüche, umfassend eine Batterie (24), die im Griffabschnitt angeordnet ist.

## Revendications

1. Dispositif de ligature pour une intervention chirurgicale, comprenant :
- un corps façonné avec un boîtier extérieur (16), le corps comprenant une portion de poignée et une portion de corps ;
- une roue rotative (4) attachée à un arbre (5) disposé à l'intérieur de la portion de corps, la roue (4) étant connectée intégralement à une gâchette (1) interposée en position adjacente à la portion de poignée, la gâchette (1) pouvant être actionnée manuellement à l'encontre de la force de tensionnement d'un ressort (3) ;
- une paire de mâchoires (12) disposées distalement sur la portion de corps et ayant une paire de crêtes (7) à leurs extrémités proximales, la paire de mâchoires (12) étant connectée à leur extrémité proximale à la portion de corps ;
- un manchon (6) connecté à l'arbre (5) et déplaçable le long de crêtes (7) lors de l'application d'une force de traction sur la gâchette (1), un commutateur de chauffage (2) prévu sur la portion de poignée, lequel, lors de l'application supplémentaire de force sur la gâchette (10), est activé de manière à déclencher le lancement, par au moins un dispositif de chauffage (13), d'une fonction de chauffage ; et
- un système de préhension comprenant : une paire d'éléments de préhension (10), un élément pousseur (14) et un élément formant cale (15), l'élément pousseur (14) étant configuré de manière à se déplacer le long de la mâchoire (12) sous l'effet d'une force appliquée à la gâchette (1) à l'encontre de l'action d'au moins un ressort à lame (11), les éléments de préhension (10) étant configurés pour recevoir au moins deux matériaux prédécoupés (9) qui, lors de l'application de chaleur par l'au moins un dispositif de chauffage (13), forment un ligateur et le ligateur étant fusionné et déployé par l'application d'une pression supplémentaire sur la gâchette.

2. Dispositif selon la revendication 1, dans lequel, lorsque le ligateur est formé de billettes sous forme de barre, le dispositif comprend en outre une chambre d'activation (20) pour convertir la barre solide en un état semi-solide.

3. Dispositif selon la revendication 1 ou 2, dans lequel une cavité est prévue sur les mâchoires (12), et dans lequel un isolant (19) est prévu autour du dispositif de chauffage (13).

4. Dispositif selon la revendication 1, dans lequel, lorsque le ligateur est formé d'un matériau en bande continue (21), au moins deux rouleaux (22) étant prévus à l'intérieur de la portion de corps pour permettre l'avance du matériau en bande continue (21).

5. Dispositif selon la revendication 1 ou 4, dans lequel les mâchoires (12) sont pourvues d'une cavité pour permettre la formation du ligateur hors du matériau en bande (12) suite au chauffage par les dispositifs de chauffage (13).

6. Dispositif selon l'une quelconque des revendications précédentes, comprenant un moyen d'alimentation électrique (8) pour l'alimentation de puissance à l'au moins un dispositif de chauffage (13).

7. Dispositif selon l'une quelconque des revendications précédentes, comprenant une batterie (24) disposée à l'intérieur de la portion de poignée.
